Europäisches Patentamt

European Patent Office

Office européen des brevets

(11) Publication number: **0 226 465 B1**

## EUROPEAN PATENT SPECIFICATION

(45) Date of publication of patent specification: **10.03.93**

(51) Int. Cl.⁵: **G01N 33/52**, //G01N33/68, G01N33/72

(21) Application number: **86309690.5**

(22) Date of filing: **12.12.86**

(54) **Integral multilayer analytical element.**

(30) Priority: **12.12.85 JP 279859/85**
**12.12.85 JP 279860/85**
**12.12.85 JP 279861/85**
**03.03.86 JP 45596/86**

(43) Date of publication of application:
**24.06.87 Bulletin 87/26**

(45) Publication of the grant of the patent:
**10.03.93 Bulletin 93/10**

(84) Designated Contracting States:
**DE FR GB**

(56) References cited:
**EP-A- 0 166 365**
**FR-A- 2 355 290**
**US-A- 3 847 553**
**US-A- 4 132 528**
**US-A- 4 256 693**

(73) Proprietor: **FUJI PHOTO FILM CO., LTD.**
**210 Nakanuma Minami Ashigara-shi**
**Kanagawa 250-01(JP)**

(72) Inventor: **Nagatomo, Shigeru**
**c/o Fuji Photo Film Co.Ltd., 3-11-46, Senzui**
**Asaka-shi, Saitama(JP)**
Inventor: **Hiratsuka, Nobuo**
**c/o Fuji Photo Film Co.Ltd., 3-11-46, Senzui**
**Asaka-shi, Saitama(JP)**
Inventor: **Ikeda, Teppei**
**c/o Fuji Photo Film Co.Ltd., 3-11-46, Senzui**
**Asaka-shi, Saitama(JP)**

(74) Representative: **Arthur, Bryan Edward et al**
**Withers & Rogers 4 Dyer's Buildings Holborn**
**London EC1N 2JT (GB)**

**Description**

BACKGROUND OF THE INVENTION

Field of the invention

The present invention relates to an integral multilayer analytical element in the form of a flat plate or a sheet for use in the analysis (assay) of the desired analyte (substance to be analyzed) in an aqueous liquid sample, and more particularly to an integral multilayer analytical element in the form of a flat plate or a sheet for use in the analysis of an analyte such as bilirubin or protein in aqueous liquid samples, particularly body fluids such as whole blood in clinical test.

Description of prior art

As one type of dry analytical elements, there have been proposed a number of integral multilayer analytical elements (hereinafter referred to as multilayer analytical element) having such a structure that a reagent layer comprising a color-forming reagent dispersed in a hydrophilic polymer binder such as gelatin is provided on a transparent support and a porous spreading layer (hereinafter referred to as spreading layer) is provided as a uppermost layer, and they have been put to practical use. Such multilayer analytical elements are disclosed in more detail, for instance, in Japanese Patent Publication No. 53(1978)-21677, U.S. Patent No. 3,992,158, U.S. Patent Reissue No. 30267, Japanese Patent Provisional Publication Nos. 55(1980)-90859, 55(1980)-164356, 57(1982)-66359, 52(1977)-3488, 58(1983)-77661, 60(1985)-222769, 57-(1982)-148250 and 57(1982)-125847, etc. In these multilayer analytical elements, fine light-blocking particles such as fine titanium dioxide particles are incorporated into the spreading layer, or a light-blocking layer is interposed between the spreading layer and the reagent layer for the purpose of effectively analyzing analytes in whole blood samples. The multilayer analytical element allows the accurate analysis of an analyte or semiquantitative determination to be carried out by depositing only about 10 μℓ of an aqueous liquid sample such as whole blood or plasma on the spreading layer thereof and hence, the dry analytical method using the multilayer analytical element is an analytical method which has gained increasing use in the filed of clinical test.

In the above-described integral multilayer analytical elements, a hydrophilic polymer binder such as gelatin is used in layers such as the reagent layer and the light-blocking layer other than the spreading layer and hence, these layers are water-permeable. However, there is generally a disadvantage that the permeation of analyte therethrough by diffusion is slow, high-molecular analyte, lipid are hydrophobic analyte are impermeable or difficultly permeable therethrough and hence, the element cannot be utilized for the analysis of these analytes. Further, there is a disadvantage that it is impossible to use the element for the analysis of all of the analytes, when whole blood is used as the aqueous liquid sample.

To cope with the problems in the analysis of high-molecular analyte, the lipid or hydrophobic analyte, there has been proposed in Japanese Patent Publication No. 56(1981)-45999 that a color-forming reagent composition containing enzyme is incorporated in the spreading layer of the element for the measurement of cholesterol. However, the element still has a disadvantage in that the light-blocking effect on the whole blood sample is insufficient. Further, there has been proposed in Japanese Patent Provisional Publication No. 59(1984)-120957 that a porous reagent layer arranged in contact with the spreading layer is used, said porous reagent layer having such a structure that a reagent composition is contained in the voids of open-cell void-containing porous structure layer comprising fine solid particles dispersed in a hydrophilic polymer binder. The element has a disadvantage in that the reagent composition to be incorporated in the porous reagent layer is limited to certain type.

SUMMARY OF THE INVENTION

It is an object of the present invention to provide an integral multilayer analytical element which allows an analyte in a sufficient amount for color reaction to rapidly penetrate into the reagent layer.

It is another object of the present invention to provide an integral multilayer analytical element which has a function capable of separating erythrocyte in a whole blood sample and makes it possible to make the analysis of an analyte in the whole blood sample by avoiding the interference of erythrocyte in the measurement of optical reflection density by colorimetry.

It is a further object of the present invention to provide an integral multilayer analytical element suitable for use in the analysis of a high-molecular analyte, a lipid, and a hydrophobic analyte.

EP 0 226 465 B1

It is a still further object of the present invention to provide an integral multilayer analytical element suitable for use in the analysis of a protein and total proteins.

It is a still further object of the present invention to provide an integral multilayer analytical element which contains a color-forming reagent composition in a sufficient amount for a color reaction with an analyte, provides a calibration curve having a steep gradient and makes measurement to be conducted with high accuracy.

It is a still further object of the present invention to provide an integral multilayer analytical element which little causes the deterioration of a colour-forming reagent composition with time, little suffers fogging is highly sensitive to the measurement and has high measuring accuracy.

The present invention provides an improvement in an integral multilayer analytical element containing at least two layer of microporous sheets integrated (porous-bonded or microporous bonded) by closely bonding two sheets with an adhesive partially provided so as to form communicating openings which do not interfere with uniform passage of a liquid between two surfaces in contact with each other, described in Japanese Patent Application No. 59 (1984)-126267 (corresponding to US-A-5 019 347 and EPA 85 107 571.3, published as EP-A-0 166 365, a document belonging to the prior art by virtue of the provisions of Article 54(3) EPC).

There is provided by the present invention an integral multilayer analytical element comprising an uppermost fibrous porous sheet, an upper non-fibrous porous sheet, a lower non-fibrous porous sheet and a water absorbing layer containing a hydrophillic polymer on a light-transmissive, water-impermeable support, wherein said upper non-fibrous porous sheet contains no light-reflecting particles and may contain a portion of a colour-forming reagent composition; said lower non-fibrous porous sheet contains a colour-forming reagent composition; said uppermost fibrous porous sheet and said upper non-fibrous porous sheet are bonded by an adhesive which is deposited on the interface between both sheets in the form of a net, crossed lines, parallel lines, dots or sand grains; and said upper non-fibrous porous sheet and said lower fibrous porous sheet are bonded by an adhesive which is deposited on the interface between both sheets in the form of a net, crossed lines, parallel lines, dots or sand grains. The non-fibrous porous sheets may be made of a membrane filter.

The present invention further provides an integral multilayer analytical element comprising an uppermost fibrous porous sheet, an upper non-fibrous porous sheet, a lower non-fibrous porous sheet and a water absorbing layer containing an alkali-resistant hydrophillic polymer on a light-transmissive, water-impermeable support, wherein said upper non-fibrous porous sheet and said lower non-fibrous porous sheet both contain no light-reflecting particles; said water absorbing layer contains a colour-forming reagent composition which comprises a water soluble cupric salt, a copper chelating agent and an alkaline agent; said uppermost fibrous porous sheet and said upper non-fibrous porous sheet are bonded by an adhesive which is deposited on the interface between both sheets in the form of a net, crossed lines, parallel lines, dots or said sand grains; and said upper non-fibrous porous sheet and said lower non-fibrous porous sheet are bonded by an adhesive which is deposited on the interface between both sheets in the form of a net, crossed lines, parallel lines, dots or sand grains.

## BRIEF DESCRIPTION OF THE DRAWINGS

Figures 1 and 2 illustrates examples of layer constitutions of the analytical elements of the present invention.

Figures 3A, 3B and 3C show examples of the adhesive patterns arranged on the interfaces of the layers (sheets).

## DETAILED DESCRIPTION OF THE INVENTION

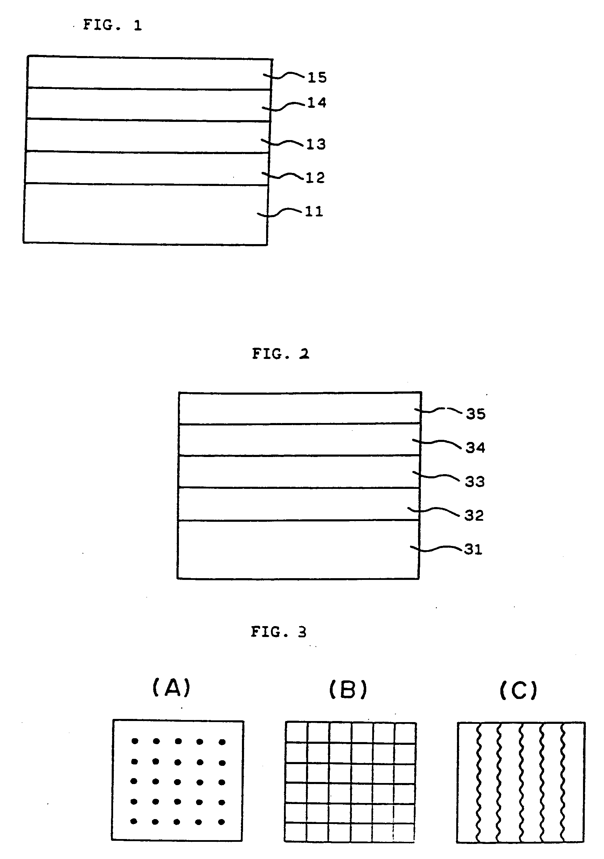

One embodiment of the multilayer analytical element of the present invention comprises a light transmissive, water-impermeable support 11, a water absorbing layer 12, a lower non-fibrous sheet 13, an upper non-fibrous porous sheet 14, and a fibrous porous sheet 15 superposed in sequence as illustrated in Figure 1.

An embodiment of the multilayer analytical element of the invention for protein analysis comprises a light-transmissive, water-impermeable support 31, a reagent layer 32, a lower non-fibrous porous sheet 33, an upper non-fibrous porous sheet 34, and a fibrous porous sheet 35 superposed in sequence as illustrated in Figure 2.

Details of the functional layers and sheets constituting the analytical element of the invention are given below.

3

The fibrous porous sheet is provided as the outermost layer (the uppermost layer which is the farthest layer from the support) and functions as a layer having a spreading effect. Namely, the sheet is a layer having a function capable of spreading an applied liquid in such a manner that the spread area of the liquid is approximately in proportion to the amount of the liquid when the liquid is applied to the surface of the upper side of the sheet (the furthermost surface from the support) as well as a function capable of supplying a uniform amount of the liquid per area to the adjoining underlayer. The sheet has a layer having a spreading effect or a metering effect. Further, the sheet functions as a layer capable of removing insoluble matters (e.g., erythrocyte in whole blood sample) by filtration, said insoluble matters interfering with the analysis of an analyte in the aqueous liquid sample.

Examples of materials for the fibrous porous sheets include woven fabric, knitted fabric, organic polymer fiber pulp-containing paper and filter paper, glass fiber pulp-containing paper and filter paper, and nonwoven fabric.

Examples of woven fabrics (woven cloth) which can be used in the present invention include those disclosed in Japanese Patent Provisional Publication Nos. 55(1980)-164356 corresponding to US-A-4 292 272 and 60(1985)-222770 corresponding to EP-A-0 162 301. Among them, plain weave fabrics disclosed in these publications as the materials for the spreading layer are preferred. Among the plain weave fabrics, thin cloth, muslin, bloadcloth and poplin are preferred. As yarns for the woven fabrics, spun yarn (twist yarn) is preferred. The yarn diameter of the woven fabric is generally in the range of about 20S to about 150S, preferably about 40 to about 120S in terms of cotton spinning yarn count or in the range of about 35 to about 300D, preferably about 45 to about 130D in terms of silk thread denier. The voids of the woven fabric are generally in the range of about 40 to about 90%, preferably about 50 to about 85%.

Examples of the knitted fabrics include warp knitted fabrics described in Japanese Patent Provisional Publication Nos. 60(1985)-222769 corresponding to EP-A-0 162 302 and 60(1985)-22770 corresponding to EP-A-0 162 301 as the material for the spreading layer, among which tricot knitted cloth, rashar knitted cloth, milanese knitted cloth and double tricot knitted cloth are preferred. As yarn for the knitted fabric, spun yarn (twist yarn) is preferred. The diameter of the yarn for the knitted fabric is generally in the range of about 40 to about 150S, preferably about 60 to about 120S in terms of cotton spinning yarn count, or in the range of about 35 to about 130D, preferably about 45D to about 90D in terms of silk thread denier. The number of knitted gauge of the knitted fabric is generally in the range of about 20 to about 50. The thickness of the knitted fabric is generally in the range of about 100 to about 600 $\mu$m, preferably about 150 to about 400 $\mu$m. The voids of the knitted fabric are generally in the range of about 40 to about 90%, preferably about 50 to about 85%.

Preferred examples of the organic polymer fiber pulp-containing paper are paper made of pulp composed of polyethylene fiber alone and paper made of mixed pulp of polyethylene fiber (30 to 70%) and natural fiber stated in Japanese Patent Provisional Publication No. 57(1982)-148250. The thickness of paper is generally in the range of about 80 to about 400 $\mu$m, preferably about 100 to about 250 $\mu$m. The voids of paper are generally in the range of about 20 to about 80%, preferably about 50 to about 70%.

Woven fabric or knitted fabric (both are hereinafter referred to as cloth) for the fibrous porous sheet is preferably a fabric from which fats and oils deposited thereon are substantially removed by degreasing treatment such as water washing when the yarn or the cloth is prepared. There are used papers or calendered filter papers on which fats and oils are substantially not deposited. The fibrous porous sheet may be processed to enhance the adhesion to an underlying layer by porous bonding, to control the metering effect or to control the metering effect so as to make it correspond to hematocrit value. For instance, one side or both sides of the sheet may be subjected to physical activating treatment (preferably glow discharge or corona discharge treatment) disclosed in Japanese Patent Provisional Publication No. 57-(1982)-66359. The sheet may be subjected to an impregnating, coating or spraying treatment with an aqueous solution of a surfactant, preferably a nonionic surfactant described in Japanese Patent Provisional Publication Nos. 55(1980)-164356, 57(1982)-66359 and 57(1982)-148250; or an impregnating, coating or spraying treatment with an aqueous solution of a hydrophilic cellulose derivative and a nonionic surfactant having an HLB value of 10 or above disclosed in Japanese Patent Provisional Publication No. 60(1985)-222770. These treatments may be used either alone or in a combination of two or more of them.

In the integral multilayer analytical element of the present invention, the upper non-fibrous porous sheet of the adjoining two non-fibrous porous sheets to be bonded by a communicating openings structure-forming adhesive layer, functions as a layer through which the liquid sample is filtered to remove insoluble matters (e.g., fine particle components such as erythrocyte and leucocyte in a whole blood sample) interfering with the analysis of the sample. The lower non-fibrous porous sheet functions as a layer having a light-blocking effect or as a porous reagent layer retaining part or the whole of the reagent composition.

EP 0 226 465 B1

As the non-fibrous porous sheet, there are used membrane filters (brush polymer layers) composed of cellulose esters such as cellulose acetate, cellulose butyrate, cellulose acetate butyrate (mixed ester) and cellulose nitrate; cellulose ethers such as ethylcellulose; carbonate polymer such as polycarbonate of bisphenol A; and polyamides such as polycapramide (nylon 6) and polyhexamethylene adipamide (nylon 6,6) described in Japanese Patent Publication No. 53(1978)-21677 corresponding to US-A-3 992 158 and U.S. Patent Nos. 3,992,158 and 1,421,341; microporous membranes composed of polyolefins such as polyethylene and polypropylene described in the publication of the Ninth Plastic Film Research Meeting Course Lecture (High-Molecular Material Associate, Feb. 22, 1984) (written in Japanese) and catalog of Membrane, Inc. (issued July 1982); communicating openings containing porous layers comprising fine particles such as polymer microbead, glass microbead and diatomaceous earth dispersed in a hydrophilic polymer binder described in Japanese Patent Publication No. 53(1978)-21677 corresponding to US-A-3 992 158 and U.S. Patent No. 3,992,158; and non-fibrous isotropic porous layers such as communicating microvoid-containing porous layers (three-dimensional lattice structure layer) comprising polymer microbeads bonded in a dotted form with a polymer adhesive which is not swollen by water, described in Japanese Patent Provisional Publication No. 55(1980)-90859, corresponding to US-A-4 258 001.

There can be used a composite membrane filter (composite brushed polymer layer) having a communicating void structure formed by closely bonding two layers of microporous high-molecular material layer at the interface therebetween, said layers being formed by a multilayer coating method or a multilayer casting method, instead of using said two non-fibrous porous sheets bonded by adhesive layers having a communicating micropore structure.

The void size of the lower non-fibrous porous sheet is generally in the range of about 20 nm to about 30 $\mu$m, preferably about 50 nm to about 10 $\mu$m. The voids thereof are generally in the range of about 20 to about 90%, preferably about 40 to about 85%, and the thickness thereof is generally in the range of about 20 to 500 $\mu$m, preferably about 80 to about 250 $\mu$m. The void size (average minimum pore size) of the upper non-fibrous porous sheet is generally in the range of about 0.8 to about 30 $\mu$m, preferably about 1.0 to about 10 $\mu$m. The voids thereof are generally in the range of about 20 to about 90%, preferably about 40 to about 85%, and the thickness thereof is generally in the range of about 20 to about 500 $\mu$m, preferably about 80 to about 350 $\mu$m. The combined thickness of the two layers of the upper and lower non-fibrous sheets is in the range of about 40 to about 500 $\mu$m, preferably about 80 to about 350 $\mu$m from the viewpoint of the measuring sensitivity.

In the multilayer analytical element o the invention, the colour-forming reagent composition is incorporated in one or more layers other than the support. Examples of the cases where two or more layers contain the color-forming reagent composition, include two layers of the hydrophilic polymer binder layer and the lower non-fibrous porous sheet layer; two layers of the hydrophilic polymer binder layer and the upper non-fibrous porous sheet layer; two layers of the two non-fibrous porous sheet layers; three layers of the hydrophilic polymer binder layer and two non-fibrous porous sheet layers; and three layers of the fibrous porous sheet layer and two non-fibrous porous sheet layers. The whole of the color-forming reagent composition may be incorporates in any combination of these two and three layers. Alternatively, each of specific ingredients may be separately incorporated in each of two or more layers to prevent functions such as sensitivity from being lowered or fogging from being caused by the interaction of the specific ingredients in the color-forming reagent composition during the course of the preparation of the element or during storage before the use of the element. In the case where the whole ingredients of substantially the same color-forming reagent composition are incorporated in two or more layers, the content of the reagent composition per unit area of each layer may be the same or different.

Part or the whole of the ingredients of the color-forming reagent composition may be incorporated in the fibrous porous sheet or two non-fibrous porous sheet layers. In such a case, it is sometimes preferred that a component containing enzyme, particularly a component containing enzyme such as oxidase or hydrolase and an ingredient capable of activating or promoting the action of enzyme is incorporated in the fibrous porous sheet or the upper non-fibrous porous sheet, because sensitivity in the measurement is heightened. Alternatively, a pretreating reagent composition such as intrinsic ammonia-trapping reagent composition described in Japanese Patent Provisional Publication No. 59(1984)-21398 and Japanese Patent Application No. 60(1985)-122348 may be incorporated in the fibrous porous sheet or the upper non-fibrous porous sheet to make it function as a pretreatment layer or a trap layer.

The incorporation of the color-forming reagent composition varies depending on an analyte in an aqueous liquid sample and a chemical reaction or a biochemical reaction catalyzed by enzyme used for the analysis of the analyte. When enzyme participates in two or more reactions, the entire ingredients of the reagent composition may be incorporated in the above-described two or three layers, or specific ingredients in the reagent composition may be separately incorporated in the two or more layers.

5

Examples of the color-forming reagent compositions containing at least one enzyme include improved Trinder reagent compositions containing glucosidase and peroxidase for use in the analysis of glucose described in U.S. Patent No. 3,992,158, Japanese Patent Publication Nos. 53 (1978)-21677 and 55(1980)-25840, Japanese Patent Provisional Publication Nos. 54(1979)-26793, 55(1980)-164356, 57(1982)-208997, 59(1984)-20853, 59(1984)-46854 and 59(1984)-54962; reagent compositions containing cholesterol oxidase, peroxidase and optionally cholesterol esterase for use in the analysis of cholesterol described in Japanese Patent Publication No. 56(1981)-45599 and Japanese Patent Provisional Publication No. 59(1984)-193352; reagent compositions containing urease for use in the analysis of blood urea nitrogen (BUN) described in Japanese Patent Provisional Publication Nos. 52(1977)-3488, 58(1983)-77661 and 56(1981)-70460; reagent compositions containing lipoprotein lipase, glycerol kinase, $\alpha$-glycerol-3-phosphoric acid oxidase and peroxidase for use in the analysis of triglyceride or glycerol described in Japanese Patent Provisional Publication No. 53(1978)-24893, etc.; reagent compositions containing bilirubin-specific oxidase and peroxidase for use in the analysis of bilirubin described in Japanese Patent Provisional Publication Nos. 54(1979)-151193 and 60(1985)-78580; reagent compositions containing uricase and peroxidase for use in the analysis of uric acid described in Japanese Patent Provisional Publication Nos. 53(1978)-26188 and 59(1984)-193352; and color-forming reagent compositions containing peroxidase for use in the detection of hydrogen peroxide described in Japanese Patent Provisional Publication Nos. 55(1980)-124499 and 58(1983)-86457.

For instance, an enzyme-containing reagent composition can be incorporated in the porous sheet before the incorporation of said sheet into the multilayer analytical element in such a manner that said sheet is immersed in an aqueous solution or organic solvent solution of the enzyme-containing reagent composition, the solution is then removed from the sheet and the sheet is dried, preferably dried under vacuum or freeze-dried under vacuum.

The enzyme-containing reagent composition can be incorporated in the porous layer after the incorporation of the layer into the multilayer analytical element in such a manner that the porous layer is provided on an adhesive layer or a water absorbing layer, and an aqueous solution or organic solvent solution of the enzyme-containing reagent composition is applied to the surface of he porous layer and the layer is dried, preferably under vacuum as described in Japanese Patent Provisional Publication Nos. 59-(1984)-171864, 60(1985)-222769 and 60(1985)-222770.

As preferred examples for separately incorporating each of specific ingredients of a color-forming reagent composition in two or more layers, there are color-forming reagent compositions for the measurement of enzyme activity or protein-bonded low-molecular analyte through reduction type coenzyme, said composition containing electron transferring agent, dye precursor, oxidation type coenzyme and enzyme capable of converting said oxidation type coenzyme into reduction type coenzyme or enzyme substrate therefor as principal ingredients. It is often preferred that each of the electron transferring agent and the dye precursor are separately incorporated in each of different two layers, because the deterioration of the color-forming reagent composition with time is reduced, fogging is reduced, the sensitivity of the measurement is increased and the accuracy of the measurement is improved (other ingredients may be used together with one of the above ingredients or may be separately incorporated in a different layer or different layers).

Examples of the color-forming reagent compositions containing the electron transferring agent, the dye precursor, the oxidation type coenzyme and the enzyme or enzyme substrate capable of converting said oxidation type coenzyme into reduction type coenzyme as principal ingredients include color-forming reagent compositions for the measurement of activity of lactate dehydrogenase described in the literature, Clinica Chimica Acta, 12, 210 (1965) and Japanese Patent Provisional Publication Nos. 59(1984)-44658 and 59(1984)-88097; color-forming reagent compositions for the measurement of activity of aspartate aminotransferase and those for the measurement of activity of alanine aminotransferase described in the literature, Clinica Chimica Acta, 28, 431 (1970) and Japanese Patent Provisional Publication Nos. 50(1975)-44894, 57-(1982)-208998, 59(1984)-44658 and 59(1984)-88097; color-forming reagent compositions for the measurement of activity of creatine kinase described in Japanese Patent Publication No. 46(1971)-9988; color-forming reagent composition for the measurement of activity of creatine phosphokinase described in Japanese Patent Provisional Publication Nos. 49(1974)-11395, 59(1984)-44658 and 59(1984)-88097; color-forming reagent compositions for the measurement of activity of testosterone and androsterone described in U.S. Patent No. 3,791,933; color-forming reagent compositions for the measurement of activity of amylase described in Japanese Patent Publication No. 56(1984)-39637; color-forming reagent compositions for the analysis of glycerol described in Japanese Patent Publication No. 53(1978)-21677; and color-forming reagent compositions for the analysis of triglyceride described in Japanese Patent Provisional Publication Nos. 50(1975)-126494 and 53(1978)-24893 and Japanese Patent Publication No. 56(1984)-38199.

In the case that the integral multilayer analytical element of the present invention is for analysis of proteins such as single protein or total proteins, the color-forming reagent composition preferably is a biuret reagent composition comprizing a water-soluble cupric salt, a copper-chelating agent and an alkaline agent. Examples of the alkaline agent include lithium hydroxide or a combination of lithium hydroxide and other alkaline compound. Examples of the biuret reagent composition include a biuret composition essentially free from sodium ion which comprises a water-soluble cupric salt (e.g., cupric sulfate), a copper-chelating agent (e.g., tartaric acid essentially free from sodium ion) and an alkaline agent essentially free from sodium ion (e.g., lithium hydroxide or potassium hydroxide), as disclosed in Japanese Patent Provisional Publication No. 54(1979)-101395; a biuret reagent composition which comprises a water-soluble cupric salt (e.g., cupric sulfate), a copper-chelating agent (e.g., tartaric acid, potassium sodium tartarate), and an alkaline agent composition comprising at least two alkaline compounds selected from the group consisting of an alkali metal hydroxide (e.g., lithium hydroxide, sodium hydroxide), an alkali metal salt (e.g., sodium chloride, sodium phosphate, sodium acetate) and an alkaline earth metal salt as disclosed in Japanese Patent Provisional Publication No. 59(1984)-51356; and a biuret reagent composition which comprises a water-soluble copper salt (e.g., copper sulfate, copper chloride, copper acetate), a copper-chelating agent (e.g., a mixture of tartaric acid and sodium tartarate), and an alkaline agent (lithium hydroxide) as disclosed in Japanese Patent Application No. 60(1985)-132057.

The amounts and ratios of the components of the biuret reagent composition are as follows. The water-soluble cupric salt is used in an amount of approx. 0.5 -10 g per 1 g of protein (analyte). The copper-chelating agent is used in a molar ratio of approx. 0.2 - 2.0 per mole of the water-soluble cupric acid. The alkaline agent is employed in an amount enough for keeping the surrounding conditions of the biuret reagent composition under analytical operation at a pH value of not lower than approx. 12.0. Lithium hydroxide is used in an amount of not less than approx. 45 wt.% (preferably approx. 50 -95 wt.%) of the total amount of the alkaline agents. The alkaline agent can be composed only of lithium hydroxide.

The color-forming reagent for the protein analysis is contained in an alkali-resistant, non-proteinaceous hydrophilic polymer binder to form a reagent layer. Examples of such non-proteinaceous polymer binder include agarose, pullulan, pulluran derivatives such as carboxymethylpullulan, polyacrylamide, polyvinylalcohol, poly-N-vinyl-2-pyrrolidone, and acrylamide-N-vinyl-2-pyrrolidone copolymer. The non-proteinaceous binder polymer can be employed singly or in combination. Preferred are agarose, polyacrylamide, polyvinyl alcohol, acrylamide-N-vinyl-2-pyrrolidone copolymer, and other alkali-resistant non-proteinoceous hydrophilic polymers.

The color-forming composition such as the biuret reagent composition can be contained in the reagent layer in an amount of approx. 10 - 50 wt.%, preferably approx. 15 - 30 wt.%, per the amount of the binder polymer. The reagent layer has a dry thickness of in the range of approx. 3 - 50 $\mu$m, preferably approx. 5 - 30 $\mu$m. The amunt of the composition for application on the support is in the range of approx. 3 - 50 g/m$^2$, preferably approx. 5 - 30 g/m$^2$. The reagent layer can contain other additives which are mentioned in this specification.

The reagent layer can be a porous layer.

When a reagent layer is provided between the lower non-fibrous porous sheet and a water absorbing layer, it is preferred that the reagent layer is a layer comprising a color-forming reagent composition dispersed in a hydrophilic polymer binder similar to those used in the water absorbing layer.

The water absorbing layer is a layer which comprises as a main component a hydrophilic polymer which swells upon the absorption of water, and functions as a layer which absorbs water in an aqueous liquid sample which reaches the surface of the water absorbing layer or penetrates therethrough. When a whole blood sample is used, said layer has a function capable of promoting the penetration of aqueous liquid components and plasma into the lower non-fibrous porous sheet (porous reagent layer). The hydrophilic polymers which can be used in the water-absorbing layer have a swelling ratio ranging from approx. 150% to approx. 2,000%, preferably from approx. 250% to approx. 1500% at 30°C when water is absorbed. Examples of such hydrophilic polymers include gelatin (e.g., acid-processed gelatin, deionized gelatin, etc.) and gelatine derivatives (e.g., phthalated gelatin, hydroxyacrylate grafted gelatin, etc.) described in Japanese Patent Provisional Publication Nos. 59(1984)-171864 and 60(1985)-115859; and agarose, pullulan, pullulan derivatives, polyacrylamide, polyvinyl alcohol and polyvinyl pyrrolidone described in Japanese Patent Provisional Publication Nos. 59(1984)-171864 and 60(1985)-115859. These hydrophilic polymers may be used either alone or as a mixture of two or more of them. Generally, gelatin and derivatives thereof are preferred. However, when analyte is albumin or other protein, it is desirable to use hydrophilic polymers such as polyacrylamide and polyvinyl alcohol other than gelatin of derived protein.

The dry thickness of the water absorbing layer is generally in the range of approx. 2 $\mu$m to approx. 100 $\mu$m, preferably approx. 5 $\mu$m to approx. 30 $\mu$m, and the water absorbing layer is coated in an amount of approx. 2 to approx. 100 g/m$^2$, preferably approx. 5 to approx. 30 g/m$^2$. The water absorbing layer may contain known pH buffering agent, organic carboxylic acid, acidic polymer, basic polymer or the like to adjust pH at the time of use (during analytical operation). Further, the water absorbing layer may contain known mordant or polymeric mordant. Though it is desirable that the water absorbing layer is transparent, a small amount of fine powder of titanium dioxide, barium sulfate or carbon black may be optionally incorporated in said layer to adjust its optical property.

As the light-transmissive, water-impermeable support of the integral multilayer analytical element of the present invention, there can be used a light-transmissive (i.e., transparent), water-impermeable support used for conventional multilayer analytical element. Examples of such supports include transparent (that is, capable of transmitting an electromagnetic wave having a wavelength in the range of approx. 200 nm to approx. 900 nm) support in the form of a film or sheet having a smooth surface and being made of a polymer such as polyethylene terephthalate, polycarbonate of bisphenol A, polystyrene and cellulose esters (e.g., cellulose diacetate, cellulose triacetate, cellulose acetate propionate, etc.). The thickness of the support is generally in the range of approx. 50 $\mu$m to approx. 1mm, preferably approx. 80 $\mu$m to approx. 300 $\mu$m. If desired, the support may contain fine powder of titanium dioxide, barium sulfate or carbon black to adjust its optical performance. There may be provided an undercoating layer (e.g. subbing layer) or an adhesive layer on the surface of the support to enhance the adhesion between the support and the water absorbing layer to be provided on the support.

In the integral multilayer analytical element of the present invention, the fibrous porous sheet and two non-fibrous porous sheets are integrated by closely bonding them to each other (by porous-bonding or microporous-bonding them to each other) with communicating microvoid (micropore) structural adhesive layers so as to allow the uniform passage of aqueous liquid to be smoothly carried out from upper side to lower side between passages formed by these sheets. The term "uniform passage of aqueous liquid" used herein means that an aqueous liquid deposited (spotted) on the surface of the upper side of the fibrous porous sheet having communicating openings is diffused by the metering action of said communicating openings and reaches the surface of the underside of said layer in a distributed state and while said distributed state is substantially kept, the aqueous liquid passes through the surface of the upper side of the adjoining upper non-fibrous porous sheet, or that the aqueous liquid which reaches the surface of the under side of the upper non-fibrous porous sheet, passes through the surface of the upper side of the adjoining non-fibrous porous sheet while the distributed state is substantially kept.

As the communicating microvoid structural adhesive layer, there are used adhesive layers wherein an adhesive is partially deposited, for instance, an adhesive layer wherein an adhesive is regularly deposited in the dotted form of circle, square, star or indeterminated form as in the spot of printing; an adhesive layer wherein an adhesive is deposited in the form of parallel fine line (corrugated or straight line) or crossed fine line (corrugated or straight line) (in this case, a communicating micropore structural adhesive layer is formed); and an adhesive layer wherein an adhesive is deposited in such a manner that the form, the size and the arrangement are microscopically random, but microscopically uniform as in screenless dot (sand grain pattern). The ratio of the gross area of the dots or lines of the adhesive per unit area, that is, the ratio of the area of the adhesive per unit area is about 80% or below, preferably about 50% or below, most preferably about 5% to about 20% in terms of the ratio of the area of net dots in printing [see, Printing Engineering Handbook, pp. 262 - 263 (1983), edited by the Printing Society of Japan. The diameter of the dot or the size of the line of the adhesive is generally in the range of about 50 $\mu$m to about 1 mm, and should be preferably small to such an extent that failure in adhesion is not caused. The space between adhesive dots or lines is in the range within which no capillary passageway is formed between the opposing surfaces of two porous sheets to be bonded to each other. The space therebetween is generally in the range of about 30 $\mu$m to about 3 mm and can be experimentally determined. For instance, two sheets of 150 $\mu$m thick membrane filters having a smooth surface are integrated by closely bonding them to each other with an adhesive deposited in the form of dots having a diameter and space within the range specified above (by porous bonding). When aqueous liquid droplets are deposited (spotted) on one surface thereof, there is substantially no difference in liquid-spread pattern and area between the droplet-deposited surface and the surface of the opposite side. In another embodiment, a 150 $\mu$m thick membrane filter having a smooth surface and a 200 $\mu$m thick tricot knitted cloth are used. An adhesive is deposited only on the elevated parts of the yarn of the tricot cloth, and the filter and the cloth are laminated to thereby effect porous bonding.

Examples of the adhesives which can be used in the present invention include liquid adhesives, non-Newtonian liquid adhesives are hot-melt adhesives. As the liquid adhesives, there are preferred adhesives having a viscosity of about 1000 mPa.s (cps) or above and exhibiting less stringing, because they can be locally deposited on the surface of the porous sheet and hardly penetrate into the interior of the sheet. Examples of such liquid adhesives include an aqueous polyvinyl alcohol solution, an aqueous dextrine solution and an aqueous solution of carboxymethylcellulose. As the non-Newtonian liquid adhesive, there

8

are preferred liquid adhesives to which non-Newtonian viscosity has been imparted by mixing fine solid particles, etc. therewith. Examples of such non-Newtonian liquid adhesives include starch paste, methylcellulose-containing starch paste and an aqueous emulsion of a vinyl acetate-butyl acrylate copolymer. As the hot-melt adhesives, there are preferred adhesives which are a solid at a temperature of about 55°C or lower. Examples of such hot-melt adhesives include an ethylenevinyl acetate copolymer and an ethylene-isobutyl acrylate copolymer. The hot-melt adhesive is used in the form of a string, a fine woolen yarn, a granule, a fine particle or a powder.

Preferred examples of methods for forming the partial patterns of the adhesive such as net dot type pattern, parallel fine line (corrugated or straight line) type pattern, crossed fine line (corrugated or straight line) type pattern and sand grain pattern are printing methods. Examples of the printing methods include a method wherein an adhesive pattern is formed directly on the surface of the porous sheet by intaglio process or gravure printing; a method wherein an adhesive pattern is transferred to rubber roller or release paper and then transferred to the surface of the porous sheet by an off-set printing system; and a method wherein an adhesive pattern is formed directly on the surface of the porous sheet through a polyethylene terephthalate gauze screen or metal screen by screen printing or said pattern is formed on the surface of the sheet by offset printing. When the hot-melt adhesive is used, these methods can be carried out at a temperature of higher than the melting point of the adhesive.

As another method for forming the partial pattern of the adhesive, there is a method wherein a liquid adhesive to which non-Newtonian viscosity has been imparted by mixing fine solid particles is applied to the surface of a temporary support to form a thin layer, and a membrane filter or paper is uniformly put thereon and then immediately peeled off to form an adhesive pattern in the form of a screenless dot in printing on the surface of the membrane filter or paper.

As still other method for forming the partial pattern of the adhesive, there is a method wherein the surface of a temporary support is coated with an adhesive to form a thin layer, a porous sheet having a regular fine uneven surface such as fabric cloth or knitted cloth is uniformly put thereon and then immediately peeled off to thereby deposit the adhesive in a dotted form only on the elevated parts of the texture (elevated parts of yarn) of the fabric cloth or knitted cloth and the porous sheet is then laminated onto other porous sheet (e.g., membrane filter) having a smooth surface to integrate them (to effect porous bonding).

As still further other method for forming the partial pattern of the adhesive, there is a method wherein the whole of the fibrous porous sheet is immersed in an adhesive solution, the sheet is taken out of the solution and pressed to squeeze the adhesive solution from the sheet and the resulting sheet in which the adhesive is deposited only on the surface of the fiber is laminated onto other porous sheet (e.g., membrane filter) having a smooth surface to integrate them (to effect porous bonding).

The porous sheet on the surface of which the partial pattern is formed, is put on other porous sheet (on the surface of which the adhesive pattern is not formed) (at a temperature of higher than the melting point of adhesive when a hot-melt adhesive is used). A substantially uniform pressure is slightly applied thereto to bond the opposing sheets to each other, whereby the porous sheets are integrated by closely bonding them to each other with a communicating microvoid (or micropore) structural adhesive layer (hereinafter referred to as porous bonding or microporous bonding). The porous bonding can be applied to bonding between porous sheets, between a porous sheet and a non-porous sheet and between non-porous sheets.

In the integral multilayer analytical element of the present invention, there may be optionally provided further additional one or more layers of fibrous porous sheet or non-fibrous porous sheet by porous bonding.

In addition, there may be optionally provided various functional layers such as indicator layer, mordant layer or detecting layer, semipermeable membrane layer, barrier layer, migration-preventing layer, pretreating or trap layer, light-blocking or light-reflecting layer (background layer), a second water-absorbing layer, etc. disclosed in the specifications of the above-described Patent Publications.

The integral multilayer analytical element of the present invention is suitable for use in the analysis of an analyte (a substance to be analyzed) in an aqueous liquid sample, for instance, high-melocular analytes such as protein, albumin and various enzyme, analytes bonded to a high-molecular weight material (protein) such as bilirubin and hydrophobic analytes such as cholesterol and triglyceride in blood, particularly whole blood as well as low-molecular weight analytes such as glucose, urea, uric acid and creatine in blood or urine. Further, the analytical element of the present invention can be used for the analysis of antibody and antigen by an immunological method, when an antibody or antigen is incorporated in the porous layer of the element. The integral multilayer analytical element of the present invention is suitable for use in the analysis of whole blood sample in particular, but can be used for the analysis of other aqueous liquid samples such as plasma, serum, urine, spinal fluid, foods, beverage, etc.

In an embodiment of the integral multilayer analytical element which is shown in Example 1 of the present invention, the element comprises (1) a fibrous porous sheet composed of tricot fabric cloth, (2) a non-fibrous porous sheet composed of cellulose acetate membrane filter, (3) a non-fibrous porous sheet composed of cellulose acetate membrane filter, (4) a water absorbing layer mainly composed of gelatin and (5) a light-transmissive, water-impermeable support in a laminated form in this order. In this element, between the porous sheets (1) and (2) and between the porous sheets (2) and (3) there are formed communicating openings by starch paste deposited in the form of net dots to thereby integrate them (to effect porous bonding). The fibrous porous sheet (1) has primarily a metering effect, the non-fibrous porous sheet (2) has primarily a blood cell-filtering effect and a light-blocking effect, the non-fibrous porous sheet (3) is a porous reagent layer which is impregnated with an enzyme-containing color-forming reagent composition and retains the same and also functions as a color forming layer, and the water absorbing layer (4) has an effect of absorbing water in a whole blood sample and promoting the transferring of aqueous liquid component to the non-fibrous porous sheet (3).

It is preferred from the viewpoints of production, packaging, transportation, storage, measuring operation, etc. that the multilayer analytical element of the present invention is cut into small pieces such as pieces about 15 to about 30 mm square or circles having approximately the same size and each piece is put into a slide frame to form a chemical analytical slide as described in Japanese Patent Publication No. 57(1982)-28331, Japanese Utility Model Provisional Publication Nos. 56(1981)-142454 and 58(1983)-32350, Japanese Patent Provisional Publication No. 57(1982)-63452 and Republished PCT Patent Application No. 58(1983)-501144. The element in the form of a long tape may be put into a cassette or magazine, or the element in the form of a small piece may be sticked on or put into a card having an opening according to the purpose of use.

The multilayer analytical element of the invention can be used for the analysis of an analyte in a liquid sample according to the disclosures of the above-described patent specifications. For instance, from about 5 to about $50\mu\ell$, preferably 8 to 30 $\mu\ell$ of an aqueous liquid such as whole blood, plasma, or serum is deposited on the spreading layer, and the element is incubated at a constant temperature of about 20 to 40°C, preferably about 37°C for one to ten minutes. Color, fluoresence or tubidity within the element, or the optical density of the reagent spreading layer is measured with a light having an ultraviolet light absorbing maximum wavelength or a light having a wavelength in the vicinity thereof from the side of the light-transmissive support by reflection photometry, and the content of the analyte in the liquid sample can be determined through the principle of colorimetry by using a calibration curve which has been previously prepared. The analysis of the analyte can be made with high accuracy, when the amount of the aqueous liquid sample to be deposited and incubation time and temperature are kept constant. The measurement can be easily made with high accuracy by using chemical analytical devices described in Japanese Patent Provisional Publication Nos. 56(1981)-77746, 58(1983)-21566 and 58(1983)-161867.

The following examples further illustrate the present invention.

Example 1 and Comparison Example 1

Integral multilayer analytical element for the analysis of total bilirubin

The surface of a 180 $\mu$m thick colorless transparent polyethylene terephthalate (PET) film (support) having a gelatin subbing layer was coated with an aqueous solution of polyvinyl alcohol having an average degree of polymerization of 1000 and a degree of saponification of about 88% in such an amount as to give a layer of 25 $\mu$m in thickness after drying, and the coated support was dried to form a water absorbing layer. The surface of the water absorbing layer was uniformly wetted with water at about 25°C, and a cellulose acetate membrane filter having the minimum pore size of 1.2 $\mu$m, a thickness of 140 $\mu$m and a void volume of about 80% as a lower non-fibrous porous sheet was then laminated on the water absorbing layer. The laminate was dried to integrate them.

Separately, there was prepared an aqueous solution of a color-forming reagent composition for the analysis of bilirubin, which contained 10 g. of dyphylline (Chemical Abstracts Registry No. 479-18-5), 2 g. of sulfosalicylic acid and 0.2 g. of 2,4-dichlorobenzenediazonium sulfosalicylate, each amount being per 100 g. of the aqueous solution. The upper side of the lower non-fibrous membrane filter was coated with said aqueous solution in an amount of 80 m$\ell$/m$^2$ and dried. In this way, the filter was impregnated with the color-forming reagent composition and retained the same.

Starch paste was deposited on the surface of the lower membrane filter through 100 mesh gauge (the ratio of the area of net dots: about 20%) by screen printing to deposit about 3 g. of past solid per m$^2$ on the surface of the filter. Immediately, a cellulose acetate membrane filter having the minimum pore size of 3.0

μm, thickness of 140 μm and a void volume of about 80% as an upper non-fibrous porous sheet was laminated thereon, and the starch paste was dried, whereby two sheets of the membrane filters was integrated by porous bonding.

In a similar manner to that described above, a tricot knitted cloth having thickness of about 250 μm composed of PET spun yarn (100S) was laminated on the upper membrane filter to integrate them by porous bonding. In this way, three layers of the porous sheets were laminated through the adhesive layers having the communicating microvoid structure (by porous bonding), whereby an integral multilayer analytical element (Example 1) for the analysis of total bilirubin could be obtained.

For the purpose of comparison, the procedure of Example 1 was repeated except (a) that the upper membrane filter was omitted and two layers of the porous sheets were bonded by porous bonding to provide an integral multilayer analytical element (Comparison Example 1A) for the analysis of total bilirubin and (b) that the tricot cloth was omitted and two layers of the porous sheets were bonded by porous bonding to provide an integral multilayer analytical element (Comparison Example 1B) for the analysis of total bilirubin.

A whole blood sample having a hematocrit value of 45% and a bilirubin content of 0.5 mg/dℓ (assayed value by Jendrassik-Grof method) was prepared. Separately, there was prepared each of control whole blood samples having a hematocrit value of 45% and a bilirubin content (assayed value) of 4.1, 8.0 or 16.2 mg/dℓ by centrifuging the above-described whole blood to separate it into plasma and blood clot, replacing part of plasma with an equal volume of control serum Omega Bilirubin (Trade name) and again mixing blood clot.

In order to evaluate the above three elements, 20 μℓ of each of the whole blood sample and the control whole blood samples was deposited on the plain weave fabric layer of each of the multilayer analytical elements of Example 1 and Comparison Examples 1A & 1B. Each element was incubated at 37°C for 6 minutes, and color formation within the element was measured with a visible light having a central wavelength of 540 nm from the side of the PET support by reflection photometry. The results are shown in Table 1. In the element of Example 1 and Comparative Example 1A, the whole blood sample was sufficiently spread. In the element of Comparison Example 1B, however, the whole blood sample was left in a droplet form which stood above the surface at the time of deposition and the spreading thereof was in sufficient.

## Table 1

| Total bilirubin content in whole blood, g/dℓ | 0.5 | 4.1 | 8.0 | 16.2 |
|---|---|---|---|---|
| Optical reflection density | | | | |
| Example 1 | 0.202 | 0.297 | 0.385 | 0.530 |
| Comp. Ex. 1A | 0.618 | 0.636 | 0.650 | 0.673 |
| Comp. Ex. 1B | 0.225 | 0.266 | 0.298 | 0.350 |

It is apparent from the results of Table 1 that since in the element of the present invention, optical reflection density in the region of low bilirubin content is low and the gradient of calibration curve is steep, bilirubin content-measurable range is wide and the measurement can be made with high accuracy. This is thought to be due to the fact that though the analyte, bilirubin sufficiently penetrates into the reagent layer, erythrocyte is removed by an upper layer before the reagent layer and hence, erythrocyte does not interfere with the measurement of optical reflection density.

In the multilayer analytical element of Comparison Example 1A having two porous layers wherein the uppermost layer is the tricot cloth layer and the next layer is the membrane filter containing the color-forming reagent composition, optical reflection density is high irrespective of the bilirubin content and the gradient of calibration curve is gentle and hence, it will be of no practical use as an analytical element for the analysis of bilirubin in whole blood. This is thought to be due to the fact that a certain amount of erythrocyte is not removed by the tricot cloth layer, reaches the membrane filter layer containing the color-forming reagent composition and interferes with the measurement of optical reflection density.

In the multilayer analytical element of Comparison Example 1B having two membrane filter layers wherein the lower membrane filter layer retains the color-forming reagent composition, the gradient of calibration curve is gentle and the analytical accuracy is low. Further, from the behavior of the whole blood sample at the time of deposition, it is apparent that it will be of no practical use as an analytical element for the analysis of total bilirubin in the whole blood sample.

Example 2

Integral multilayer analytical element for the analysis of total bilirubin

The procedure of Example 1 was repeated except that deionized gelatin and polyvinyl alcohol having an average degree of polymerization of 1000 and a degree of saponification of about 88% were used in such an amount as to give the water absorbing layer of 1, 2, 5, 10 or 25 $\mu$m in thickness after drying. There were prepared 10 kinds of integral multilayer analytical elements for the analysis of total bilirubin, wherein three layers composed of one tricot cloth layer as the uppermost layer and two membrane filter layers were laminated by porous bonding.

Whole blood samples similar to those of Example 1 were used and there were prepared calibration curves for these ten multilayer analytical elements in a similar manner to that described in Example 1. A difference in optical reflection density between total bilirubin contents 16.2 mg/d$\ell$ and 0.5 mg/d$\ell$ was determined to evaluate accuracy in the analysis of total bilirubin. The results are shown in Table 2.

## Table 2

| Thickness of water absorbing layer | 1μm | 2μm | 5μm | 10μm | 25μm |
|---|---|---|---|---|---|
| Component of water absorbing layer | difference in optical reflection density value | | | | |
| Gelatin | 0.074 | 0.175 | 0.264 | 0.295 | 0.306 |
| PVA | 0.098 | 0.202 | 0.304 | 0.321 | 0.328 |
| Evaluation | CC | BB | AA | AA | AA |

Note:  Gelatin:  deionized gelatin
         PVA:  polyvinyl alcohol
Evaluation symbol:
    AA:   The gradient of calibration curve is steep and analytical accuracy is high (similar evaluation to that of Example 1).
    BB:   The gradient of calibration curve is slightly gentler than that of AA and analytical accuracy is slightly inferior to AA.
    CC:   The gradient of calibration curve is gentle and analytical accuracy is low (similar evaluation to that of Comparison Example 1B).

It is apparent from the results of Table 2 that when the thickness of the water absorbing layer is only 1 μm, the gradient of calibration curve is gentle and analytical accuracy is low, even when the layer is composed of both gelatin and polyvinyl alcohol. It is also apparent that when the thickness of the layer is 2 μm, the gradient of calibration curve is slightly gentler than those of the 5 - 25 μm thick layers and the 2 μm thick layer is in analytical accuracy slightly inferior to the 5 - 25 μm thick layers, but is usable; and when the thickness if 5 to 25 μm, the gradient of calibration curve is steep, analytical accuracy is high and these layers are usable. Accordingly, it is clear that the thickness of the water absorbing layer must be at least 2 μm, preferably at least 5 μm.

Example 3

Integral multilayer analytical element for the analysis of albumin

The surface of a 180 $\mu$m thick colorless transparent PET film having gelatin subbing layer was coated with an aqueous solution of polyvinyl alcohol having an average degree of polymerization of 1000 and a degree of saponification of about 88% in such an amount as to give a layer of 25 $\mu$m in thickness after drying, and the coated film was dried to form a water absorbing layer. The surface of the water absorbing layer was uniformly wetted with water at about 25°C, and a cellulose acetate membrane filter having the minimum pore size of 1.2 $\mu$m, a thickness of 140 $\mu$m and a void volume of about 80% as a lower non-fibrous porous sheet was laminated thereon. The laminate was dried to integrate them.

Separately, there was prepared a solution of a color-forming reagent composition for the analysis of albumin, which contained 0.5 g. of Bromocresol Green and 0.37 g. of maleic acid, each amount being per 100 g. of a mixed solvent of water and methanol (1:3 by weight). The upper side of the lower membrane filter was coated with said solvent solution in an amount of 130 m$\ell$/m$^2$ and dried. In this way, the filter was impregnated with the color-forming reagent composition and retained the same.

Starch paste was deposited on the surface of the lower membrane filter through 100 mesh gauge (the ratio of the area of net dots being about 20%) by screen printing to deposit about 3 g. of paste solid per m$^2$ on the surface of the filter. Immediately, a cellulose acetate membrane film having the minimum pore size of 3.0 $\mu$m, a thickness of 140 $\mu$m and a void volume of about 80% as an upper non-fibrous porous sheet was laminated thereon, and the starch paste was dried to integrate two sheets of the membrane filters by porous bonding.

Similarly, a plain weave fabric having a thickness of about 140 $\mu$m composed of PET spun yarn (100S) was laminated on the upper membrane filter to integrate them by porous bonding. In this way, there was prepared an integral multilayer analytical element for the analysis of albumin, wherein three layers of the porous sheets were laminated by porous bonding.

A whole blood sample having a hematocrit value of 42% and an albumin content of 4.2 g/d$\ell$ (assayed value) was prepared. Separately, there were prepared control whole blood samples having a hematocrit value of 42% and albumin contents (assayed value) of 1.4, 7.7 and 10.0 g/d$\ell$ by centrifuging the aforementioned whole blood to separate it into plasma and blood clot, replacing part of plasma with an equal volume of physiological saline or 15% human albumin-containing physiological saline and again mixing the separated blood clot.

20 $\mu\ell$ of each of the whole blood sample and the control whole blood samples was deposited on the plain weave fabric layer of each analytical element, and the element was incubated at 37°C for 6 minutes. Color formation within the element was measured with a visible light having a central wavelength of 640 nm from the side of the PET support by reflection photometry. The results are shown in Table 3.

It is apparent from the results of Table 3 that in the element of the present invention, the gradient of calibration curve is steep and analytical accuracy is high. This is thought to be due to the fact that a sufficient amount of the analyte albumin in the whole blood sample rapidly penetrates into the porous reagent layer and hence, a color reaction proceeds well.

14

Table 3

| Albumin content in whole blood sample, g/dl | 1.4 | 4.2 | 7.7 | 10.0 |
|---|---|---|---|---|
| Optical reflection density | 0.401 | 0.674 | 0.908 | 1.020 |

Example 4

Integral multilayer analytical element for the analysis of albumin

The procedure of Example 3 was repeated except that Bromocresol Purple was used in place of Bromocresol Green. There was prepared an integral multilayer analytical element for the analysis of albumin, wherein three layers of the porous sheets were bonded by porous bonding.

In a similar manner to that described in Example 3, 20 $\mu l$ of each of the whole blood sample and the control whole blood samples were deposited on the plain weave fabric layer of the analytical element, and the element was incubated at 37°C for 6 minutes. Color formation within the element was measured with a visible light having a central wavelength of 600 nm from the side of PET support by reflection photometry. The results are shown in Table 4.

Table 4

| Albumin content in whole blood sample, g./dl | 1.4 | 4.2 | 7.7 | 10.0 |
|---|---|---|---|---|
| Optical reflection density | 0.365 | 0.601 | 0.833 | 0.946 |

It is apparent from the results of Table 4 that in the analytical element for the analysis of albumin, which contains Bromocresol Purple as a color indicator, the gradient of calibration curve is steep and accuracy for the analysis of albumin is high. This is thought to be due to the fact that a sufficient amount of the analyte albumin in the whole blood sample quickly penetrates into the porous reagent layer and hence, a color reaction proceeds well.

Example 5

Integral multilayer analytical element for the analysis of total bilirubin

The surface of a 180 $\mu$m thick colorless transparent polyethylene terephthalate (PET) film (support) having a gelatin subbing layer was coated with an aqueous solution of polyvinyl alcohol having an average degree of polymerization of 1000 and a degree of saponification of about 88% in such an amount as to give a layer of 25 $\mu$m in thickness after drying, and the coating support was dried to form a water absorbing layer. The surface of the water absorbing layer was uniformly wetted with water at about 25°C, and a cellulose acetate membrane filter having the minimum pore size of 1.2 $\mu$m, a thickness of 140 $\mu$m and a void volume of about 80% as a lower non-fibrous porous sheet was then laminated on the water absorbing layer. The laminate was dried to integrate them.

Separately, there was prepared an aqueous solution of color-forming reagent composition for the analysis of bilirubin, which contained 10 g. of dyphylline (Chemical Abstracts Registry No. 479-18-5), 2 g. of sulfosalicylic acid and 0.2 g. of 2,4-dichlorobenzenediazonium sulfosalicylate, each amount being per 100 g. of the aqueous solution. The upper side of the lower non-fibrous membrane filter was coated with said aqueous solution in an amount of 80 m$\ell$/m$^2$ and dried. In this way, the filter was impregnated with the color-forming reagent composition and retained the same. Thus, a first reagent-containing porous layer was prepared.

Another cellulose acetate membrane filter having the minimum pore size of 3.0 $\mu$m, thickness of 140 $\mu$m and a void volume of about 80% was dipped in the above-produced aqueous solution of color-forming reagent composition to introduce the solution into the pores of the membrane filter, and thus treated membrane filter was taken out of the solution and kept to dryness. Thus, another reagent-containing membrane filter for forming the second reagent-containing porous layer was prepared.

Starch paste was deposited on the surface of the lower membrane filter through 100 mesh gauge (the ratio of the area of net dots: about 20%) by screen printing to deposit about 3 g. of past solid per m$^2$ on the surface of the filter. Immediately, the above reagent-containing membrane filter was laminated on the lower membrane filter (i.e., first reagent-containing porous layer), and the starch paste was dried, whereby two sheets of the reagent-containing membrane filters were integrated by porous bonding.

In a similar manner to that described above, a tricot knitted cloth having thickness of about 250 $\mu$m composed of PET spun yarn (100S) was laminated on the upper membrane filter to integrate them by porous bonding. In this way, three layers of the porous sheets were laminated through the adhesive layers having the communicating microvoid structure (by porous bonding), whereby an integral multilayer analytical element for the analysis of total bilirubin could be obtained.

A whole blood sample having a hematocrit value of 45% and a bilirubin content of 0.5 mg/d$\ell$ (assayed value by Jendrassik-Grof method) was prepared. Separately, there was prepared each of control whole blood samples having a hematocrit value of 45% and a bilirubin content (assayed value) of 4.1, 8.0 or 16.2 mg/d$\ell$ by centrifuging the above-described whole blood to separate it into plasma and blood clot, replacing part of plasma with an equal volume of control serum Omega Bilirubin (Trade name) and again mixing blood clot.

In order to evaluate the analytical element, 20$\mu\ell$ of each of the whole blood sample and the control whole blood samples were deposited on the plain weave fabric layer of the multilayer analytical element. Each element was incubated at 37°C for 6 minutes, and color formation within the element was measured with a visible light having a central wavelength of 540 nm from the side of the PET support by reflection photometry. The results are shown in Table 5. In the element of Example 5, the whole blood sample was sufficiently spread.

For the purpose of comparison, the aforementioned results of Comparison Example 1 are also set forth in Table 5.

## Table 5

| | | | | |
|---|---|---|---|---|
| Total bilirubin content in whole blood, g/dℓ | 0.5 | 4.1 | 8.0 | 16.2 |
| | | | | |
| Optical reflection density | | | | |
| Example 5 | 0.213 | 0.330 | 0.451 | 0.620 |
| Comp. Ex. 1A | 0.618 | 0.636 | 0.650 | 0.673 |
| Comp. Ex. 1B | 0.225 | 0.266 | 0.298 | 0.350 |

It is apparent from the results of Table 5 that since in the analytical element of the present invention having two reagent-containing porous layers, optical reflection density in the region of low bilirubin content is low and the gradient of calibration curve is steep, bilirubin content-measurable range is wide and the measurement can be made with high accuracy. This is thought to be due to the fact that though the analyte, bilirubin sufficiently penetrates into the reagent layer, erythrocyte is removed by an upper layer before the reagent layer and hence, erythrocyte does not interfere with the measurement of optical reflection density.

Further, the same analytical procedure was repeated ten times on the analytical elements prepared in the same manner according to Example 5. There was observed little variation among the measured optical density values. Hence, it was confirmed that the analytical element of Example 5 shows satisfactory reproduceability.

Example 6

Integral multilayer analytical element for the analysis of albumin

The surface of a 180 $\mu$m thick colorless transparent PET film having gelatin subbing layer was coated with an aqueous solution of polyvinyl alcohol having an average degree of polymerization of 1000 and a degree of saponification of about 88% in such an amount as to give a layer of 25 $\mu$m in thickness after drying, and the coated film was dried to form a water absorbing layer.

Separately, there was prepared a solution of a color-forming reagent composition for the analysis of albumin, which contained 0.5 g. of Bromocresol Green and 0.37 g. of maleic acid, each amount being per 100 g. of a mixed solvent of water and methanol (1:3 by weight). This color-forming reagent composition solution was applied to the surface of the above-prepared water absorbing layer in an amount of 50 mℓ/m$^2$, and dried. Thus, a first color-forming reagent composition-containing layer was prepared.

The surface of the first reagent-containing layer was uniformly wetted with water at about 25°C, and a cellulose acetate membrane filter having the minimum pore size of 1.2 $\mu$m, a thickness of 140 $\mu$m and a void volume of about 80% as a lower non-fibrous porous sheet was laminated thereon. The laminate was dried to integrate them.

The upper side of the lower membrane filter was coated with the above solution in an amount of 130 mℓ/m$^2$ and dried. In this way, the filter was impregnated with the color-forming reagent composition and retained the same. Thus, a second color-forming reagent composition-containing layer (porous layer) was prepared.

Starch paste was deposited on the surface of the lower membrane filter through 100 mesh gauge (the ratio of the area of net dots being about 20%) by screen printing to deposite about 3 g. of paste solid per m² on the surface of the filter. Immediately, a cellulose acetate membrane film having the minimum pore size of 3.0 μm, a thickness of 140 μm and a void volume of about 80% as an upper non-fibrous porous sheet was laminated thereon, and the starch paste was dried to integrate two sheets of the membrane filters by porous bonding.

Similarly, a plain weave fabric having a thickness of about 140 μm composed of PET spun yarn (100S) was laminated on the upper membrane filter to integrate them by porous bonding. In this way, there was prepared an integral multilayer analytical element for the analysis of albumin, wherein three layers of the porous sheets were laminated by porous bonding.

A whole blood sample having a hematocrit value of 42% and an albumin content of 4.2 g/dℓ (assayed value) was prepared. Separately, there were prepared control whole blood samples having a hematocrit value of 42% and albumin contents (assayed value) of 1.4, 7.7 and 10.0 g/dℓ by centrifuging the aforementioned whole blood to separate it into plasma and blood clot, replacing part of plasma with an equal volume of physiological saline or 15% human albumin-containing physiological saline and again mixing the separated blood clot.

20 μℓ of each of the whole blood sample and the control whole blood samples was deposited on the plain weave fabric layer of each analytical element, and the element was incubated at 37°C for 6 minutes. Color formation within the element was measured with a visible light having a central wavelength of 640 nm from the side of the PET support by reflection photometry. The results are shown in Table 6.

It is apparent from the results of Table 6 that in the element of the present invention, the gradient of calibration curve is steep and analytical accuracy is high. This is thought to be due to the fact that a sufficient amount of the analyte albumin in the whole blood sample rapidly penetrates into the porous reagent layer and hence, a color reaction proceeds well.

## Table 6

| Albumin content in whole blood sample, g/dℓ | 1.4 | 4.2 | 7.7 | 10.0 |
|---|---|---|---|---|
| Optical reflection density | 0.483 | 0.840 | 1.193 | 1.356 |

Example 7

Integral multilayer analytical element for the analysis of albumin

The procedure of Example 6 was repeated except that Bromocresol Purple was used in place of Bromocresol Green. There was prepared an integral multilayer analytical element for the analysis of albumin, wherein three layers of the porous sheets were bonded by porous bonding.

In a similar manner to that described in Example 6, 20 μℓ of each of the whole blood sample and the control whole blood samples was deposited on the plain weave fabric layer of the analytical element, and the element was incubated at 37°C for 6 minutes. Color formation within the element was measured with a visible light having a central wavelength of 600 nm from the side of PET support by reflection photometry. The results are almost the same as those of Eample 6.

18

This is thought to be due to the fact that a sufficient amount of the analyte albumin in the whole blood sample quickly penetrates into the porous reagent layer and hence, a color reaction proceeds well.

Example 8

Integral multilayer analytical element for the analysis of total bilirubin

There was prepared an aqueous solution of a color-forming reagent composition for the analysis of bilirubin, which contained 10 g. of dyphylline (Chemical Abstracts Registry No. 479-18-5), 2 g. of sulfosalicylic acid and 0.2 g. of 2,4-dichlorobenzenediazonium sulfosalicylate, each amount being per 100 g. of the aqueous solution.

In the obtained aqueous solution was dipped a cellulose acetate membrane filter having the minimum pore size of 1.2 $\mu$m, a thickness of 140 $\mu$m and a void volume of about 80% to introduce the solution within the pores of the filter, and dried to produce a reagent-containing porous sheet.

Starch paste was deposited on the surface of the membrane filter (the above reagent-containing porous sheet) through 100 mesh gauge (the ratio of the area of net dots: about 20%) by screen printing to deposit about 3 g. of past solid per m$^2$ on the surface of the filter. Immediately, a cellulose acetate membrane filter having the minimum pore size of 3.0 $\mu$m, thickness of 140 $\mu$m and a void volume of about 80% as an upper-fibrous porous sheet was laminated thereon, and the starch paste was dried, whereby two sheets of the membrane filters were integrated by porous bonding.

In a similar manner to that described above, a tricot knitted cloth having thickness of about 250 $\mu$m composed of PET spun yarn (100S) was laminated on the upper membrane filter to integrate them by porous bonding. In this way, three layers of the porous sheets were laminated through the adhesive layers having the communicating microvoid structure (by porous bonding), whereby an integral multilayer analytical element for the analysis of total bilirubin could be obtained.

A whole blood sample having a hematocrit value of 45% and a bilirubin content of 0.5 mg/d$\ell$ (assayed value by Jendrassik-Grof method) was prepared. Separately, there was prepared each of control whole blood samples having a hematocrit value of 45% and a bilirubin content (assayed value) of 4.1, 8.0 or 16.2 mg/d$\ell$ by centrifuging the above-described whole blood to separate it into plasma and blood clot, replacing part of plasma with an equal volume of control serum Omega Bilirubin (Trade name) and again mixing blood clot.

In order to evaluate the obtained analytical element, 20 $\mu\ell$ of each of the whole blood sample and the control whole blood samples was deposited on the plain weave fabric layer of the multilayer analytical element. The element was incubated at 37°C for 6 minutes, and color formation within the element was measured with a visible light having a central wavelength of 540 nm from the side of the PET support by reflection photometry. The results are shown in Table 7. In the element of Example 8 the whole blood sample was sufficiently spread.

## Table 7

| Total bilirubin content in whole blood, g/d$\ell$ | 0.5 | 4.1 | 8.0 | 16.2 |
|---|---|---|---|---|
| Optical reflection density | | | | |
| Example 8 | 0.284 | 0.362 | 0.440 | 0.556 |

It is apparent from the results of Table 7 that since in the element of the present invention, optical reflection density in the region of low bilirubin content is low and the gradient of calibration curve is steep, bilirubin content-measurable range is wide and the measurement can be made with high accuracy. This is thought to be due to the fact that though the analyte, bilirubin sufficiently penetrates into the reagent layer, erythrocyte is removed by an upper layer before the reagent layer and hence, erythrocyte does not interfere with the measurement of optical reflection density.

Example 9

Integral multilayer analytical element for the analysis of total proteins

On a colorless transparent PET support of 180 $\mu$m thick (the surface ofwhich had been treated with glow discharge) was coated the aqueous solution of a reagent composition for measurement of total protein content having the below-mentioned composition to give a dry layer of 40 $\mu$m thick, and then the coated reagent layer was dried.

Composition of reagent composition for measurement of totan protein content.

| Water | 60 g. |
|---|---|
| Sodium polyoxydiethylenelauryl-sulfate (50% aqueous solution) | 40 g. |
| Tartaric acid | 100 g. |
| Copper sulfate 5 hydrates | 150 g. |
| Sodium tartarate | 15 g. |
| Lithium hydroxide monohydrate | 200 g. |
| Acrylamide/N-vinylpyrrolidone (1:1) copolymer 20 % aqueous solution (viscosity (40°C): approx. 400 cps) | 2000 g. |

The surface of the dried reagent layer was uniformly wetted with water at about 25°C, and a cellulose acetate membrane filter having the minimum pore size of 1.2 $\mu$m, a thickness of 140 $\mu$m and a void volume of about 80% as a lower non-fibrous porous sheet was then laminated on the reagent layer. The laminate was dried to integrate them.

Starch paste was deposited on the surface of the membrane filter (the above porous sheet) through 100 mesh gauge (the ratio of the area of net dots: about 20%) by screen printing to deposit about 3 g. of past solid per m$^2$ on the surface of the filter. Immediately, a cellulose acetate membrane filter having the minimum pore size set forth in Table 8, thickness of 140 $\mu$m and a void volume of about 80% as an upper non-fibrous porous sheet was laminated thereon, and the starch paste was dried, whereby two sheets of the membrane filters were integrated by porous bonding.

In a similar manner to that described above, a plain weave cloth having thickness of about 140 $\mu$m composed of PET spun yarn (100S) was laminated on the upper membrane filter to integrate them by porous bonding. In this way, five analytical elements for quantitative measurement of total protein content in which three layers of the porous sheets were laminated through the adhesive layers having the communicating microvoid structure (by porous bonding) and the three porous layers were arranged on the reagent-containing hydrophilic polymer layer coated on a support.

A whole blood sample having a hematocrit value of 43% and a total protein content (of plasma) of 7.2 g/d$\ell$ was prepared. There was prepared each of control whole blood samples having a hematocrit value of 43% and a total protein content (of plasma) of 1.8 g/d$\ell$ and 10.0 g/d$\ell$ by centrifuging the above-described whole blood to separate it into plasma and blood clot, replacing part of plasma with an equal volume of physiological saline solution or 15 % human albumin-containing physiological saline solution.

In order to evaluate the obtained analytical element, 20 $\mu\ell$ of each of the whole blood sample and the control whole blood samples was deposited on the plain weave fabric layer of the multilayer analytical element. The element was incubated at 37°C for 6 minutes, and color formation within the element was measured with a visible light having a central wavelength of 540 nm from the side of the PET support by reflection photometry.

The results are shown in Table 8.

Table 8

### Minimum pore size (µm) of non-fibrous porous sheets

| | | | | | |
|---|---|---|---|---|---|
| Upper sheet | 0.45 | 0.8 | 1.2 | 3.0 | 5.0 |
| Lower sheet | 3.0 | 3.0 | 3.0 | 3.0 | 3.0 |
| $\triangle$ OD | 0.094 | 0.347 | 0.466 | 0.502 | 0.307 |
| Evaluation | CC | BB | AA | AA | AA |

Evaluation symbol:

AA:  The gradient of calibration curve is steep and analytical accuracy is high (similar evaluation to that of Example 1).

BB:  The gradient of calibration curve is slightly gentler than that of AA and analytical accuracy is slightly inferior to AA.

CC:  The gradient of calibration curve is gentle and analytical accuracy is low (similar evaluation to that of Comparison Example 1B).

It is understood from the results of Table 8 that:

when the minimum pore size of the upper non-fibrous porous sheet is 0.45 µm, the gradient of calibration curve is gentle and the analytical accuracy is low;

when the minimum pore size of the upper non-fibrous porous sheet is 0.8 µm, the gradient of calibration curve is still gentle as compared with the case using the upper non-fibrous porous sheet having the minimum pore size in the range of 1.2 - 5.0 µm and the analytical accuracy is slightly low, but this analytical element is practically employable; and

when the minimum pore size of the upper non-fibrous porous sheet is in the range of 1.2 - 5.0 µm, the gradient of calibration curve is steep and the analytical accuracy is high, and hence this analytical element is satisfactorily employable.

Accordingly, it is apparent that in the multilayer analytical element of the type of the present invention the upper non-fibrous porous sheet preferably has the minimum pore size of not less than 0.8 µm, more preferably not less than 1.2.

Example 10

Integral multilayer analytical element for the analysis of total protein

The procedures of Example 9 were repeated except that a cellulose acetate membrane filter having the minimum pore size 0.45 $\mu$m or 1.2 $\mu$m was employed as the lower non-fibrous porous sheet. Thus, ten analytical elements for quantitative analysis of total protein (two types of lower non-fibrous porous sheets x five types of upper non-fibrous porous sheets) were prepared.

These analytical elements were subjected to the evaluation mentioned in Example 9, and gave almost the same analytical results as the corresponding results in Example 9. Accordingly, it has been confirmed that the pore size of the upper non-fibrous pore sheet defines the analytical accuracy of the analtical element.

Example 11

Integral multilayer analytical element for the analysis of total proteins

On a colorless transparent PET support of 180 $\mu$m thick (the surface ofwhich had been treated with glow discharge) was coated the aqueous solution of a reagent composition for measurement of total protein content having the below-mentioned composition to give a dry layer of 40 $\mu$m thick, and then the coated reagent layer was dried.

Composition of reagent composition for measurement of totan protein content.

| Water | 60 g. |
|---|---|
| Sodium polyoxydiethylenelauryl-sulfate (50% aqueous solution) | 40 g. |
| Tartaric acid | 100 g. |
| Copper sulfate 5 hydrates | 150 g. |
| Sodium tartarate | 15 g. |
| Lithium hydroxide monohydrate | 200 g. |
| Acrylamide/N-vinylpyrrolidone (1:1) copolymer 20 % aqueous solution (viscosity (40°C): approx. 400 cps) | 2000 g. |

The surface of the dried reagent layer was uniformly wetted with water at about 25°C, and a cellulose acetate membrane filter having the minimum pore size of 1.2 $\mu$m, a thickness of 140 $\mu$m and a void volume of about 80% as a lower non-fibrous porous sheet was then laminated on the reagent layer. The laminate was dried to integrate them.

Starch paste was deposited on the surface of the membrane filter (the above porous sheet) through 50 mesh gauge (the ratio of the area of net dots: about 20%) by screen printing to deposit about 3 g. of past solid per m$^2$ on the surface of the filter. Immediately, a cellulose acetate membrane filter having the minimum pore size of 3.0 $\mu$m, a thickness of 140 $\mu$m and a void volume of about 80% as an upper non-fibrous porous sheet was laminated thereon, and the starch paste was dried, whereby two sheets of the membrane filters were integrated by porous bonding.

In a similar manner to that described above, a tricot knitted cloth having thickness of about 200 $\mu$m composed of PET spun yarn (100S) was laminated on the upper membrane filter to integrate them by porous bonding. In this way, five analytical elements for quantitative measurement of total protein content in which three layers of the porous sheets were laminated through the adhesive layers having the communicating microvoid structure (by porous bonding) and the three porous layers were arranged on the reagent-containing hydrophilic polymer layer coated on a support.

A whole blood sample having a hematocrit value of 43% and a total protein content (of plasma) of 7.2 g/d$\ell$ was prepared. There was prepared each of control whole blood samples having a hematocrit value of 43% and a total protein content (of plasma) of 1.8, 3.3, 5.7, 8.5, or 10.0 g/d$\ell$ by centrifuging the above-described whole blood to separate it into plasma and blood clot, replacing part of plasma with an equal volume of physiological saline solution or 15 % human albumin-containing physiological saline solution.

In order to evaluate the obtained analytical element, 20 $\mu\ell$ of each of the whole blood sample and the control whole blood samples was deposited on the plain weave fabric layer of the multilayer analytical element. The element was incubated at 37°C for 6 minutes, and color formation within the element was measured with a visible light having a central wavelength of 540 nm from the side of the PET support by reflection photometry.

The results are shown in Table 9.

## Table 9

| Total protein content (g/d$l$) | 1.8 | 3.3 | 5.7 | 7.2 | 8.5 | 10.0 |
|---|---|---|---|---|---|---|
| Reflection optica density | 0.910 | 1.029 | 1.234 | 1.308 | 1.358 | 1.412 |

From the results of Table 9$\beta$, it is apparent that the multilayer analytical element of the present invention gives high measurement accuracy.

## Claims

1. An integral multilayer analytical element comprising an uppermost fibrous porous sheet (15), an upper non-fibrous porous sheet (14), a lower non-fibrous porous sheet (13) and a water absorbing layer (12) containing a hydrophilic polymer on a light-transmissive , water-impermeable support (11), wherein:

   said upper non-fibrous porous sheet (14) contains no light-reflecting particles and may contain a portion of a colour-forming reagent composition;

   said lower non-fibrous sheet (13) contains a colour forming reagent composition;

   said uppermost fibrous porous sheet (15) and said upper non-fibrous porous sheet (14) are bonded by an adhesive which is deposited on the interface between both sheets in the form of a net, crossed lines, parallel lines, dots or sand grains; and

   said upper non-fibrous porous sheet (14) and said lower non-fibrous porous sheet (13) are bonded by an adhesive which is deposited on the interface between both sheets in the form of a net, crossed lines, parallel lines, dots or sand grains.

2. The integral multilayer analytical element defined in claim 1, wherein said non-fibrous porous sheets (13, 14) are made of a membrane filter.

3. An integral multilayer analytical element comprising an uppermost fibrous porous sheet (35), an upper non-fibrous porous sheet (34), a lower non-fibrous porous sheet (33) and a water absorbing layer (32) containing an alkali-resistant hydrophilic polymer on a light-transmissive, water-impermeable support (31), wherein:

   said upper non-fibrous porous sheet (34) and said lower non-fibrous porous sheet (33) both contain no light-reflecting particles;

   said water absorbing layer (32) contains a colour-forming reagent composition which comprises a water soluble cupric salt, a copper chelating agent and an alkaline agent;

   said uppermost fibrous porous sheet (35) and said upper non-fibrous porous sheet (34) are bonded by an adhesive which is deposited on the interface between both sheets in the form of a net, crossed lines, parallel lines, dots or sand grains; and

   said upper non-fibrous sheet (34) and said lower non-fibrous porous sheet (33) are bonded by an adhesive which is deposited on the interface between both sheets in the form of a net, crossed lines, parallel lines, dots or sand grains.

4. The integral multilayer analytical element defined in claim 3, wherein said non-fibrous porous sheets (33, 34) are made of a membrane filter.

**Patentansprüche**

1. Integrales mehrschichtiges analytisches Element, dadurch **gekennzeichnet,** daß es eine oberste faserige poröse Folie (15), eine obere nicht-faserige poröse Folie (14), eine untere nicht-faserige poröse Folie (13) und eine Wasserabsorptionsschicht (12), die ein hydrophiles Polymeres enthält, auf einem lichtdurchlässigen wasserundurchlässigen Träger (11) umfaßt, wobei

die genannte obere nicht-faserige poröse Folie (14) keine lichtreflektierenden Teilchen enthält und einen Teil einer farbbildenden Reagenszusammensetzung enthalten kann;

die genannte untere nicht-faserige Folie (13) eine farbbildende Reagenszusammensetzung enthält;

die genannte oberste faserige poröse Folie (15) und die genannte obere nicht-faserige poröse Folie (14) durch einen Klebstoff, der auf der Grenzfläche zwischen beiden Folien in Form eines Netzes, sich kreuzender Linien, paralleler Linien, Punkten oder Sandkörnern aufgebracht ist, aneinander gebunden sind; und

die genannte obere nicht-faserige poröse Folie (14) und die genannte untere nicht-faserige poröse Folie (13) durch einen Klebstoff, der auf der Grenzfläche zwischen beiden Folien in Form eines Netzes, sich kreuzender Linien, paralleler Linien, Punkten oder Sandkörnern aufgebracht ist, aneinander gebunden sind.

2. Integrales mehrschichtiges analytisches Element nach Anspruch 1, dadurch **gekennzeichnet,** daß die genannten nicht-faserigen porösen Folien (13, 14) aus einem Membranfilter hergestellt sind.

3. Integrales mehrschichtiges analytisches Element, dadurch **gekennzeichnet,** daß es eine oberste faserige poröse Folie (35), eine obere nicht-faserige poröse Folie (34), eine untere nicht-faserige poröse Folie (33) und eine Wasserabsorptionsschicht (32), die ein alkalibeständiges hydrophiles Polymeres enthält, auf einem lichtdurchlässigen wasserundurchlässigen Träger (31) umfaßt, wobei

die genannte obere nicht-faserige poröse Folie (34) und die genannte untere nicht-faserige poröse Folie (33) beide keine lichtreflektierenden Teilchen enthalten,

die genannte Wasserabsorptionsschicht (32) eine farbbildende Reagenszusammensetzung, die ein wasserlösliches Kupfer(II)-Salz, ein Kupferchelatisierungsmittel und ein alkalisches Mittel umfaßt, enthält;

die genannte oberste faserige poröse Folie (35) und die genannte obere nicht-faserige poröse Folie (34) durch einen Klebstoff, der auf der Grenzfläche zwischen beiden Folien in Form eines Netzes, sich kreuzender Linien, paralleler Linien, Punkten oder Sandkörnern aufgebracht ist, aneinander gebunden sind; und

die genannte obere nicht-faserige Folie (34) und die genannte untere nicht-faserige poröse Folie (33) durch einen Klebstoff, der auf der Grenzfläche zwischen beiden Folien in Form eines Netzes, sich kreuzender Linien, paralleler Linien, Punkten oder Sandkörnern aufgebracht ist, aneinander gebunden sind.

4. Integrales mehrschichtiges analytisches Element nach Anspruch 3, dadurch **gekennzeichnet,** daß die genannten nicht-faserigen porösen Folien (33, 34) aus einem Membranfilter hergestellt sind.

**Revendications**

1. Un élément intégral multicouche pour analyse comprenant une feuille poreuse fibreuse la plus élevée (15), une feuille poreuse non fibreuse supérieure (14), une feuille poreuse non fibreuse inférieure (13) et une couche absorbant l'eau (12) contenant un polymère hydrophile sur un support imperméable à l'eau, transmettant la lumière (11), dans lequel :

ladite feuille poreuse non fibreuse supérieure (14) ne contient pas de particules réfléchissant à la lumière et peut contenir une portion d'une composition de réactif chromogène;

ladite feuille non fibreuse inférieure (13) contient une composition de réactif chromogène;

ladite feuille poreuse fibreuse la plus élevée (15) et ladite feuille poreuse non fibreuse supérieure (14) sont collées par un adhésif qui est déposé sur l'interface entre les deux feuilles sous la forme d'un réseau, de lignes croisées, de lignes parallèles, de points ou de grains de sable ; et

ladite feuille poreuse non fibreuse supérieure (14) et ladite feuille poreuse non fibreuse inférieure

24

(13) sont collées par un adhésif qui est déposé sur l'interface entre les deux feuilles sous la forme d'un réseau, de lignes croisées, de lignes parallèles, de points ou de grains de sable.

2. L'élément intégral multicouche pour analyse selon la revendication 1, dans lequel lesdites feuilles poreuses non fibreuses (13, 14) sont faites d'une membrane filtrante.

3. Un élément intégral multicouche pour analyse comprenant une feuille poreuse fibreuse la plus élevée (35), une feuille poreuse non fibreuse supérieure (34), une feuille poreuse non fibreuse inférieure (33) et une couche absorbant l'eau (32) contenant un polymère hydrophile résistant aux alcalis sur un support imperméable à l'eau, transmettant la lumière (31), dans lequel :

   ladite feuille poreuse non fibreuse supérieure (34) et ladite feuille poreuse non fibreuse inférieure (33) toutes deux ne contiennent pas de particules réfléchissant à la lumière;

   ladite couche absorbant l'eau (32) contient une composition de réactif chromogène qui comprend un sel cuivrique soluble dans l'eau, un agent chélatant du cuivre et un agent alcalin;

   ladite feuille poreuse fibreuse la plus élevée (35) et ladite feuille poreuse non fibreuse supérieure (34) sont collées par un adhésif qui est déposé sur l'interface entre les deux feuilles sous la forme d'un réseau, de lignes croisées, de lignes parallèles, de points ou de grains de sable; et

   ladite feuille poreuse non fibreuse supérieure (34) et ladite feuille poreuse non fibreuse inférieure (33) sont collées par un adhésif qui est déposé sur l'interface entre les deux feuilles sous la forme d'un réseau, de lignes croisées, de lignes parallèles, de points ou de grains de sable.

4. L'élément intégral multicouche pour analyse selon la revendication 3, dans lequel lesdites feuilles poreuses non fibreuses (33, 34) sont faites d'une membrane filtrante.

FIG. 1

~15
~14
~13
~12
~11

FIG. 2

~35
~34
~33
~32
~31

FIG. 3

(A)          (B)          (C)